Europäisches Patentamt

European Patent Office

Office européen des brevets

(19)

(11) Veröffentlichungsnummer : **0 449 798 A2**

(12)                    **EUROPÄISCHE PATENTANMELDUNG**

(21) Anmeldenummer : 91890039.0

(22) Anmeldetag : 26.02.91

(51) Int. Cl.⁵ : **G01N 21/64, G01N 21/78**

(30) Priorität : 27.03.90 AT 713/90

(43) Veröffentlichungstag der Anmeldung :
**02.10.91 Patentblatt 91/40**

(84) Benannte Vertragsstaaten :
**DE FR GB**

(71) Anmelder : **AVL Medical Instruments AG**
**Stettemerstrasse 28**
**CH-8207 Schaffhausen (CH)**

(72) Erfinder : **Wolfbeis, Otto S., Prof. Dr.**
**Im Hoffeld 32**
**A-8046 Graz (AT)**
Erfinder : **List, Helmut, Dipl.-Ing.**
**Bogengasse 36**
**A-8010 Graz (AT)**

(74) Vertreter : **Krause, Walter, Dr. Dipl.-Ing. et al**
**Postfach 200 Singerstrasse 8**
**A-1014 Wien (AT)**

(54) **Verfahren zur Qualitätskontrolle von verpackten, organischen Stoffen, sowie ein Verpackungsmaterial zur Durchführung des Verfahrens.**

(57)    Zur Qualitätskontrolle von verpackten, organischen Stoffen, vorzugsweise von verpackten
Lebensmitteln, Genußmitteln und Medikamenten werden die zu untersuchenden Stoffe mit
einem flächenförmigen optischen Sensorelement in kontakt gebracht, welches an der Innenseite der Verpackung angebracht ist und
auf die Änderung der Gaszusammensetzung im
Gasraum über der Probe mit einer Änderung
der Farbe oder Fluoreszenz reagiert. Die
Änderung einer der optischen Eigenschaften
des Sensorelementes wird visuell oder optoelektronisch detektiert.

EP 0 449 798 A2

Die Erfindung betrifft ein Verfahren zur Qualitätskontrolle von verpackten, organischen Stoffen, vorzugsweise von verpackten Lebensmitteln, Genußmitteln und Medikamenten, sowie ein vorteilhaftes Verpackungsmaterial zur Durchführung des Verfahrens.

Während bei unverpackten Nahrungsmitteln, Genußmitteln und Getränken eine z.B. durch Bakterien verursachte Zersetzung durch Probennahme oder den entstehenden Geruch leicht nachweisbar ist, ist dies bei verpackten Lebensmitteln nur schwer möglich, da die gasförmigen Produkte der bakteriellen Zersetzung, welche den Geruch hervorrufen, nicht durch das Verpackungsmaterial dringen können. Bei den Geruchsstoffen handelt es sich im wesentlichen um Schwefelwasserstoff, Ammoniak, organische Sulfide (Mercaptane) und organische Amine (z. B. Putrescin, Cadaverin). Gleichzeitig tritt, bedingt durch die Aktivität aerober Bakterien, eine Erniedrigung des Sauerstoffpartialdruckes und eine Erhöhung des $CO_2$-Partialdrucks ein.

Um eine Bildung der oben genannten bakteriellen Stoffwechselprodukte nachzuweisen, was es bisher erforderlich, die Packung zu durchstechen und den Gasraum über der Probe mit Hilfe entsprechender Sensoren für Sauerstoff, $CO_2$, und eventuell anderer Gase zu untersuchen. Als Sensoren können z.B. amperometrische Sauerstoffsensoren oder potentiometrische $CO_2$-Sensoren Verwendung finden.

Der Nachteil dieser Verfahren besteht allerdings darin, daß man zu deren Durchführung jeweils ein entsprechendes Probennahme- bzw. Auswertegerät braucht, daß weiters das Verpackungsmaterial durchstochen werden muß, sodaß das Lebensmittel nach der Untersuchung nicht mehr steril ist. Weiters erfordert die Durchführung eine gewisse Vertrautheit mit der Technik, da bei sorglosem Öffnen der Verpackung ein schneller Gasaustausch die Ergebnisse verfälschen kann.

In ähnlicher Weise geben verpackte Medikamente und Arzneistoffe aber auch Kosmetika bei ihrer bakteriellen oder nichtbakteriellen, z.B. hydrolytischen Zersetzung gewisse Gase ab oder verbrauchen ein Gas, wie z.B. Sauerstoff oder Kohlendioxid. Solche Zersetzungen können von außen kaum erkannt werden, zumal es sich oft um sehr geringe Substanzmengen handelt, welche gebildet oder verbraucht werden.

In diesem Zusammenhang ist beispielsweise aus der US-PS 4 746 616 ein Farbindikator bekannt geworden, welcher Lebensmitteln, Medikamenten oder Diätprodukten beigegeben werden kann und bei Kontakt mit giftigen Verunreinigungen oder Beimengungen, welche fahrlässig oder absichtlich in die Verpackung gelangen, mit einer detektierbaren Farbänderung reagiert. Beispielsweise kann der ungiftige Farbindikator an der Innenseite eines Verschlusses einer Medikamentenkapsel angeordnet sein und bei Kontakt, beispielsweise mit Cyaniden, eine Farbänderung zeigen. Mit diesem Verfahren ist es jedoch nicht möglich, ein Verderben bzw. durch Bakterien verursachtes Zersetzen organischer Stoffe nachzuweisen, da in diesem Fall keine Beimengungen oder Verunreinigungen vorliegen.

Weiters ist aus der US-PS 3 198 163 ein Feuchtigkeitsindikator bekannt, welcher an der Innenseite einer Verpackung für Produkte angeordnet ist, deren Qualität durch zu hohe Feuchtigkeit beeinträchtigt werden könnte. Das Überschreiten eines bestimmten Feuchtigkeitspegels wird durch einen von außen erkennbaren bzw. detektierbaren Farbumschlag des Indikators angezeigt.

Ähnlich funktioniert auch ein aus der US-PS 2 762 711 bekannter Tausensor, mit welchem festgestellt werden kann, ob eingefrorene Lebensmittel kurzfristig aufgetaut und dann wieder eingefroren wurden. Dabei wird in der Verpackung eines gefrorenen Produktes ein bei Kontakt mit flüssigem Wasser mit einer Farbänderung reagierender Indikator so angebracht, daß Tauwasser zum Indikator gelangen kann. Dieser zeigt dann eine irreversible Farbänderung. Beginnendes oder bereits fortgeschrittenes Verderben von verpackten, organischen Stoffen kann jedoch mit diesen Maßnahmen nicht festgestellt werden.

Aufgabe der vorliegenden Erfindung ist es, ein Verfahren zur Verfügung zu stellen, welches technisch einfach durchgeführt werden kann und ein Öffnen der Verpackung überflüssig macht. Weiters soll ein vorteilhaftes Verpackungmaterial zur Durchführung des Verfahrens angegeben werden.

Diese Aufgabe wird erfindungsgemäß dadurch gelöst, daß der organische Stoff zusammen mit einem optischen Sensorelement gasdicht eingeschlossen und so mit der Gasphase zwischen organischem Stoff und Verpackung in Kontakt gebracht wird, wobei eine auf Zersetzung des organischen Stoffes beruhende Änderung der Zusammensetzung der Gasphase zu einer Änderung einer optischen Eigenschaft, vorzugsweise einer Änderung der Farbe oder der Fluoreszenz des Sensorelementes führt, sowie daß die Änderung dieser optischen Eigenschaft visuell oder optoelektronisch detektiert wird.

Dabei wird erfindungsgemäß entweder die Zunahme des $CO_2$-, $H_2S$-, Mercaptan-, Ammoniak- oder Amin-Gehaltes oder die Abnahme des $O_2$-Gehaltes der Gasphase gemessen.

Das erfindungsgemäße Verfahren beruht somit auf der Verwendung von optischen Sensoren, welche an der Innenseite der Verpackung angebracht sind und somit in ständigem Kontakt mit der Gasphase über der Probe, oder mit ihr selbst, stehen. Diese Sensoren sind als flächige Elemente ausgebildet und reagieren auf die Bildung von $CO_2$ sowie von Geruchsstoffen aus der Gruppe der Sulfide bzw. Amine mit einer visuell oder instrumentell erkennbaren Änderung der Eigenfarbe oder -fluoreszenz. In ähnlicher Weise können an der Innenseite Sensorelemente angebracht werden, welche den Verbrauch von Sauerstoff anzeigen.

Bei einem Verpackungsmaterial zur Durchführrung des Verfahrens, welches an der einem zu verpackenden, organischen Stoff zugewandten Innenseite ein flächenförmiges, optisches Sensorelement aufweist, wobei das Verpackungsmaterial zumindest im Bereich des Sensorelementes für die visuell oder optoelektrisch zu detektierende Strahlung durchlässig ist, ist erfindungsgemäß vorgesehen, daß das Sensorelement in der Gasphase über dem organischen Stoff angeordnet ist und eine auf eine Änderung der Gaszusammensetzung innerhalb der Verpackung mit einer Änderung ihrer optischen Eigenschaften reagierende Indikatorsubstanz aufweist. Im einfachsten Ausführungsfall kann damit die Bestimmung der Farbänderung rein visuell - also ohne jedes Auswertegerät - erfolgen.

Dem Verpackungsmaterial samt optischem Sensor kann eine optische Auswerteeinheit zugeordnet sein, welche über vorzugsweise faseroptische Lichtleiter mit dem Sensorelement in Kontakt bringbar ist, wobei die Auswerteeinheit eine die Änderung der Farbe oder der Fluoreszenz des Sensorelementes anzeigende Einrichtung aufweist. Falls das Sensorelement einen Fluoreszenzindikator enthält, wird durch die optische Auswerteeinheit auch die notwendige Anregungsstrahlung zur Verfügung gestellt.

Um zu verhindern, daß nichtgasförmige Stoffe, welche in frischen Nahrungsmitteln vorhanden sind, bereits zu einer Änderung der Farbe oder Fluoreszenz der Sensorfläche führen, ist es entsprechend einer Weiterbildung der Erfindung zweckmäßig, wenn die Indikatorsubstanz in einer Indikatorschicht angeordnet ist, welche an der dem organischen Stoff zugewandten Seite mit einer gaspermeablen, hydrophoben Polymerschicht abgedeckt ist, bzw. wenn das Sensorelement aus einer gaspermeablen, hydrophoben Polymerschicht besteht, welche die Indikatorsubstanz als tröpfchenförmige wäßrige oder organischwäßrige Emulsion enthält. Die notwendige Selektivität für die zu detektierenden gasförmigen oder in der Gasphase als Dampf vorliegenden Stoffe wird vorzugsweise durch organische Polymere, wie z.B. Silicone, erreicht.

Erfindungsgemäß ist es weiters vorgesehen, daß sich zwischen Verpackungsmaterial und flächenförmigem Sensorelement eine Haftschicht befindet, wobei die dem Sensorelement zugewandte Innenseite des Verpackungsmaterials in diesem Bereich ggf. geätzt oder chemisch modifiziert ist, um die Haftfähigkeit des Sensorelementes zu erhöhen.

Um Farbänderungen des Sensorelementes bzw. der Indikatorschicht besser feststellen zu können, kann die Indikatorschicht oder die Polymerschicht vorzugsweise weiß pigmentiert sein. Farbänderungen sind dadurch trotz Eigenfarbe einer Probe (z.B. einer Fleischprobe) gut erkennbar.

Schließlich ist erfindungsgemäß vorgesehen, daß das Verpackungsmaterial in den an das Sensorelement angrenzenden Bereichen Referenzflächen bestimmter Farbe aufweist, die durch visuellen Vergleich mit dem Sensorelement Auskunft über den Frischezustand des verpackten, organischen Stoffes geben. Beispielsweise können Referenzstreifen verwendet werden, welche aus einer indikatorfreien, pigmentierten Polymerschicht bestehen, welche sich bei einem Farbumschlag der Indikatorschicht besonders deutlich von dieser abheben.

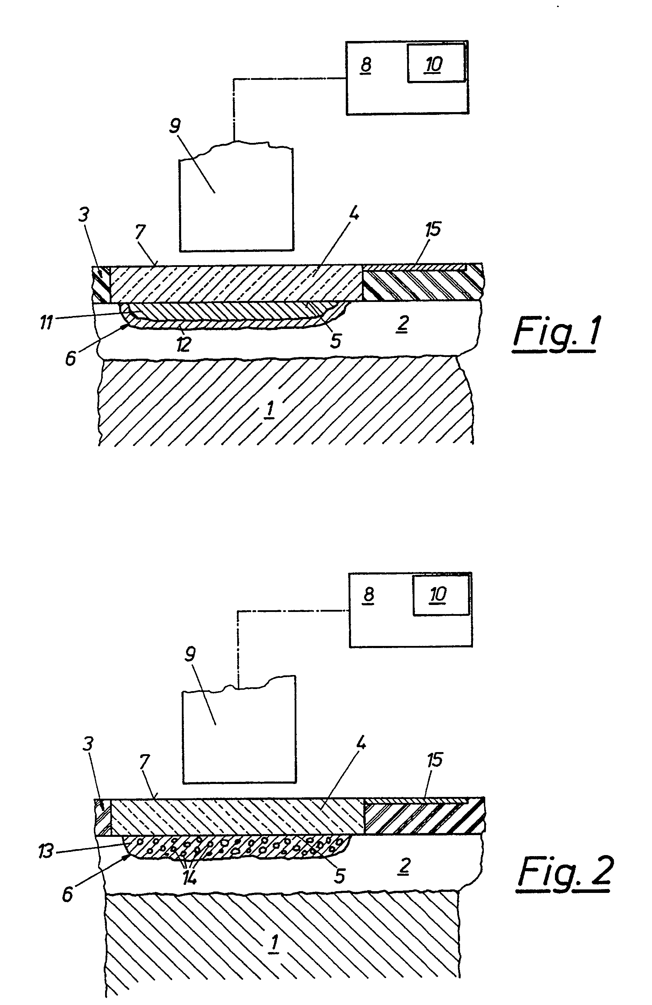

Die Erfindung wird im folgenden anhand von Zeichnungen näher erläutert. Es zeigen Fig. 1 eine erfindungsgemäße Vorrichtung mit einem erfindungsgemäßen Verpackungsmaterial und Fig. 2 eine Ausführungsvariante nach Fig. 1.

In beiden Figuren ist jeweils der organische Stoff bzw. das zu verpackende Lebensmittel mit 1 bezeichnet, der Gasraum darüber mit 2 und das Verpackungsmaterial mit 3. In einem für die zu messende Strahlung durchlässigen Bereich 4 des Verpackungsmaterials 3 ist an dessen Innseite 5 ein flächenförmiges, optisches Sensorelement 6 angeordnet, welches zum Teil am Lebensmittel 1 anliegen und unterschiedlich strukturiert sein kann. An der dem Sensorelement 6 abgewandten Seite 7 erfolgt entweder die visuelle Kontrolle der Farbänderung oder es ist eine optische Auswerteeinheit 8 vorgesehen, welche z.B. über einen faseroptischen Lichtleiter 9 mit dem Sensorelement 6 in optischem Kontakt steht. Die Auswerteeinheit 9 weist eine die Änderung der Farbe oder der Fluoreszenz anzeigende Einrichtung 10 auf. Bei Verwendung eines Fluoreszenzindikators im Sensorelement 6 kann die notwendige Anregungsstrahlung beispielsweise über einen zweiarmigen Lichtleiter zugeführt werden.

Die Vorrichtung bzw. das Verpackungsmaterial nach Fig. 1 weist ein Sensorelement 6 auf, dessen Indikatorschicht 11 mit einer gaspermeablen, hydrophoben Polymerschicht 12 abgedeckt ist. Das im organischen Stoff 1 durch bakterielle Zersetzung gebildete Gas gelangt durch die Polymerschicht 12 zur Indikatorschicht 11, in welcher es eine Änderung der Farbe oder Fluoreszenz verursacht.

In der Vorrichtung nach Fig. 2 besteht das Sensorelement 6 aus einer gaspermeablen, hydrophoben Polymerschicht 13, welche die Indikatorsubstanz als tröpfchenförmige Emulsion 14 enthält. Die eigentliche Reaktionsphase besteht hier aus einer Emulsion einer Lösung von chromogenen, fluorogenen, oder in ihrer Fluoreszenz löschbaren Farbstoffen, in einem Lösungsmittel, welches mit dem polymeren Material der Polymerschicht 13 nicht mischbar ist. Die Bildung oder der Verbrauch von gasförmigen Stoffen verursacht darin eine Änderung der Farbe oder Fluoreszenz, welche auch hier entweder rein visuell oder mit Hilfe der Faseroptik 9 und einer entsprechenden Auswerteeinheit 8 erfaßt werden kann.

Typische Schichtdicken der Sensorelemente nach Fig. 1 und 2 betragen zwischen 20 und 500 µm.

Weiters kann das Verpackungsmaterial 3 in den an das Sensorelement 6 angrenzenden Bereichen 15 Farbflächen aufweisen. Je nach farblicher Übereinstimmung dieser Flächen mit der Farbe des Sensorelementes 6 kann verdorbene von frischer Ware unterschieden werden.

Beispiele:

1) Verpackungsmaterial mit einer Emulsionsschicht zum optischen Nachweis der Bildung von Schwefelwasserstoff.

20 g SiliconBasis PE 1055A (Petrarch Systems Silanes & Silicones, ABCR GmbH, D-7500 Karlsruhe, BRD; alle weiteren Silicone wurden ebenfalls von dieser Fa. bezogen) sowie 6 µl Polymerisationsinhibitor PS 925 werden vermischt und auf ca. 0°C gekühlt. Dazu gibt man in kleinen Portionen 4 g einer 40%igen Lösung von Blei(II)nitrat in Wasser, welche auch noch 34 mg Natriumlaurylsulfat enthält und in welche 2 % Titandioxid emulgiert worden sind. Mit einem Hochgeschwindigkeitsrührer (Virtis Typ 23, The Virtis Co., Gardiner, New York 12525) wird bei Stufe 10 bis 15 15 s. lang eine Grobemulsion bereitet. Nach beendeter Zugabe wird diese durch Rühren bei Stufe 30 bis 40 zu einer feinen Emulsion verarbeitet.

Die so erhaltene Emulsion wird nun mit einer Härtungskomponente versetzt und dadurch polymerisiert. Dazu gibt man bei Raumtemperatur 2 g des Siliconhärters PE 1055B mit einer Pasteurpipette zur obigen feinen Emulsion, streicht das Material in gewünschter Größe und Dicke auf das Verpackungsmaterial, und läßt entweder 24 Stunden bei Raumtemperatur oder 2 Stunden bei 50°C polymerisieren. Man erhält eine weiße Sensorfläche, welche in Kontakt mit Schwefelwasserstoff ($H_2S$) zuerst graubraun und dann schwarz wird. Der Farbumschlag ist besonders gut erkennbar, wenn man neben der Sensormembran eine zweite Membran anbringt, welche durch Suspendieren von Titandioxid in den oben angeführten Siliconen hergestellt werden kann. Da in diesem Fall wegen des Fehlens der Bleinitratemulsion keine Farbänderung auftreten kann, wird das Erkennen auch kleinster Farbunterschiede sehr erleichtert. Anstelle von Bleinitrat können natürlich auch andere Schwermetallsalze in wäßrigen Lösungen verwendet werden (z.B. Quecksilber- und Cadmiumsalze).

2) Verpackungsmaterial mit einer Membran zum optischen Nachweis der Bildung von Ammoniak und Aminen.

Wenn in dem in Beispiel 1 angeführten Herstellungsverfahren die 40%ige Lösung von Blei(II)nitrat in Wasser durch eine Lösung von 5 mg Carboxy-SNARF-X (Produkt Nr. C-1281, Molecular Probes Inc., Eugene, Oregon) in 10 ml Bicarbonatpuffer von pH 7,0 umgesetzt wird, erhält man eine Membranemulsion, deren Farbe bei Begasen mit Ammoniak oder verdampfbaren Aminen von rotviolett nach blau umschlägt. Weiters können Farbstoffe aus der Gruppe der Naphthorhodamine bzw. Naphthofluoresceine (z.B. Carboxy-Naphthofluoresceine) verwendet werden.

3) Verpackungsmaterial mit einer Membran zum optischen Nachweis der Bildung von $CO_2$.

Man legt feine Polyacrylamidteilchen (Teilchengröße 2 bis 50 µm) in eine konzentrierte Lösung von Bromthymolblau in einem 1:1-Gemisch aus Bicarbonat-Phosphat-Puffer (pH 8,2 bis 8,5) und Methanol. Nach 6 Stunden werden die Teilchen herausgenommen, getrocknet, und in dünner Lage auf eine dünne Schicht eines Haftvermittlers (z.B. Kaschierkleber, Prod.Nr. 46.960 von Whittaker Chemicals; Brüssel, Belgien), auf dem Verpackungsmaterial aufgebracht. Bei 85°C verkrallen sich diese Teilchen innerhalb von 5 min in dieser Schicht. Danach läßt man bei Raumtemperatur stehen, drückt die Teilchen mit sanftem Druck an, und deckt sie mit einer dünnen (12 µm) Membran eines $CO_2$-permeablen Polymers ( z. B. ein Silicon-Carbonat-Copolymer, ein Polyurethan, oder ein Silicon) ab. Der Rand der Abdeckung sollte über den blauen Rand der Sensorfläche hinausgehen, um eine Randversiegelung zu gewährleisten. Nach 24-stündigem Einlegen dieser Flächen in Wasser erhält man so ein flächiges Sensorelement nach Fig. 1, welches bei Begasen mit $CO_2$ von blau über grün nach gelb umschlägt. Anstelle von Bromthymolblau kann - zum Nachweis von Ammoniak - auf den Polyacrylamidteilchen auch Carboxy-SNARF-X covalent immobilisiert werden.

In ähnlicher Weise kann man eine Vielzahl von Farbnachweisreaktionen, wie sie in der Literatur für verschiedene Gase beschrieben sind, zur Qualitätskontrolle heranziehen, indem man entweder wäßrige Emulsionen der entsprechenden Reagentien als Membranmaterialien verwendet (Fig. 1), oder die Reagentien auf einem festen Träger immobilisiert, entsprechend Beispiel 3 mit einer gaspermeablen Polymerschicht abdeckt und flächig auf das Verpackungsmaterial aufbringt (Fig. 2).

## Patentansprüche

1. Verfahren zur Qualitätskontrolle von verpackten, organischen Stoffen, vorzugsweise von verpackten Lebensmitteln, Genußmitteln und Medikamenten, **dadurch gekennzeichnet,** daß der organische Stoff zusammen mit einem optischen Sensorelement gasdicht eingeschlossen und so mit der Gasphase zwischen organischem Stoff und Verpackung in kontakt gebracht wird, wobei eine auf Zersetzung des organischen Stoffes beruhende Änderung der Zusammensetzung der Gasphase zu einer Änderung einer optischen Eigenschaft, vorzugsweise einer Änderung der Farbe oder der Fluoreszenz des Sensorelementes führt, sowie daß die Änderung dieser optischen Eigenschaft visuell oder optoelektronisch detektiert wird.

2. Verfahren nach Anspruch 1, **dadurch gekennzeichnet**, daß die Zunahme des $CO_2$-, $H_2S$-, Mercaptan-, Ammoniak- oder Amin-Gehaltes der Gasphase gemessen wird.

3. Verfahren nach Anspruch 1, **dadurch gekennzeichnet**, daß die Abnahme des $O_2$-Gehaltes der Gasphase gemessen wird.

4. Verpackungsmaterial, welches an der einem zu verpackenden, organischen Stoff (1) zugewandten Innenseite (5) ein flächenförmiges, optisches Sensorelement (6) aufweist, wobei das Verpackungsmaterial (3) zumindest im Bereich des Sensorelementes (6) für die visuell oder optoelektronisch zu detektierende Strahlung durchlässig ist, **dadurch gekennzeichnet**, daß das Sensorelement (6) in der Gasphase über dem organischen Stoff (1) angeordnet ist und eine auf eine Änderung der Gaszusammensetzung innerhalb der Verpackung mit einer Änderung ihrer optischen Eigenschaften reagierende Indikatorsubstanz aufweist.

5. Verpackungsmaterial nach Anspruch 4, **dadurch gekennzeichnet**, daß die Indikatorsubstanz in einer Indikatorschicht (11) angeordnet ist, welche an der dem organischen Stoff (1) zugewandten Seite mit einer gaspermeablen, hydrophoben Polymerschicht (12) abgedeckt ist.

6. Verpackungsmaterial nach Anspruch 5, **dadurch gekennzeichnet**, daß die Indikatorschicht (11) aus Polyacrylamidteilchen besteht, in welchen die Indikatorsubstanz, beispielsweise Bromthymolblau zum Nachweis von $CO_2$, in einer Pufferlösung vorliegt.

7. Verpackungsmaterial nach Anspruch 5, **dadurch gekennzeichnet**, daß die Indikatorschicht (11) aus Polyacrylamidteilchen besteht, auf welche die Indikatorsubstanz, beispielsweise Carboxy-SNARF-X zum Nachweis von Ammoniak, covalent immobilisiert ist.

8. Verpackungsmaterial nach Anspruch 4, **dadurch gekennzeichnet**, daß das Sensorelement (6) aus einer gaspermeablen, hydrophoben Polymerschicht (13) besteht, welche die Indikatorsubstanz als tröpfchenförmige wäßrige oder organischwäßrige Emulsion (14) enthält.

9. Verpackungsmaterial nach Anspruch 8, **dadurch gekennzeichnet**, daß die Indikatorsubstanz zum Nachweis von $H_2S$ eine wäßrige Lösung eines Schwermetallsalzes, vorzugsweise Blei(II)nitrat, enthält.

10. Verpackungsmaterial nach Anspruch 8, **dadurch gekennzeichnet**, daß die Indikatorsubstanz zum Nachweis von Ammoniak und Aminen Carboxy-SNARF-X oder Carboxy-Naphthofluoresceine enthält.

11. Verpackungsmaterial nach einem der Ansprüche 4 bis 10, **dadurch gekennzeichnet**, daß sich zwischen Verpackungsmaterial (3) und flächenförmigem Sensorelement (6) eine Haftschicht befindet, wobei die dem Sensorelement (6) zugewandte Innenseite (5) des Verpackungsmaterials in diesem Bereich ggf. geätzt oder chemisch modifiziert ist.

12. Verpackungsmaterial nach einem der Ansprüche 5 bis 11, **dadurch gekennzeichnet**, daß die Indikatorschicht (11) oder die Polymerschicht (12) vorzugsweise weiß pigmentiert ist.

13. Verpackungsmaterial nach einem der Ansprüche 5 bis 12, **dadurch gekennzeichnet**, daß das Verpackungsmaterial (3) in den an das Sensorelement (6) angrenzenden Bereichen (15) Referenzflächen bestimmter Farbe aufweist, die durch visuellen Vergleich mit dem Sensorelement (6) Auskunft über den Frischezustand des verpackten, organischen Stoffes geben.

_Fig.1_

_Fig.2_